# EUROPÄISCHE PATENTSCHRIFT

(11) **EP 2 934 419 B1**
(45) Veröffentlichungstag und Bekanntmachung des Hinweises auf die Patenterteilung: **25.05.2016**
(21) Anmeldenummer: 13820725.3
(22) Anmeldetag: 12.12.2013
(51) Int. Cl.: A61H 39/00, A61N 1/05, A61N 1/36

(54) **ELEKTRISCHES STIMULATIONSGERÄT**
ELECTRICAL STIMULATION DEVICE
APPAREIL DE STIMULATION ÉLECTRIQUE

(30) Priorität: 20.12.2012 AT 501272012
(43) Veröffentlichungstag der Anmeldung: 28.10.2015
(73) Patentinhaber: Ducest Medical GmbH, 7222 Rohrbach bei Mattersburg (AT); Biegler GmbH, 3001 Mauerbach (AT)
(72) Erfinder: SCHNETZ, Guntram, A-2362 Biedermannsdorf (AT); NETAUSCHEK, Friedrich, A-3421 Höflein an der Donau (AT)
(74) Vertreter: Sonn & Partner Patentanwälte
(86) Internationale Anmeldenummer: PCT/AT2013/050250
(87) Internationale Veröffentlichungsnummer: WO 2014/094021

(56) Entgegenhaltungen:
- EP-A1- 2 168 630
- WO-A1-2012/000003
- US-A1- 2008 288 016
- US-B1- 6 564 102

## Beschreibung

Die Erfindung betrifft ein elektrisches Stimulationsgerät mit einem Stimulator, enthaltend einen Generator zur Erzeugung elektrischer Stimulationsimpulse mit bestimmten Stimulationsparametern, eine Spannungsversorgung zur Versorgung des Generators mit elektrischer Energie und ein Stimulatorgehäuse, mit einem Befestigungselement zur Befestigung des Stimulators an der Hautoberfläche, mit zumindest zwei Nadelelektroden zum Einstechen in die Hautoberfläche eines zu stimulierenden Areals, mit einer Leitung, über welche die Nadelelektroden mit dem Stimulator verbunden sind, wobei der Stimulator lösbar mit dem Befestigungselement verbindbar ist.

Das betreffende elektrische Stimulationsgerät ist insbesondere zur Elektroakupunkturtherapie bei Menschen, aber auch Tieren anwendbar. Insbesondere ist das Gerät für den Einsatz der elektrischen Stimulation im Bereich des Ohres geeignet. Anwendungsgebiete reichen von der Schmerztherapie über die Wundheilung bis hin zur Therapie von Durchblutungsstörungen, beispielsweise bei Diabetikern.

Ein elektrisches Stimulationsgerät der gegenständlichen Art ist beispielsweise aus der WO 2012/000003 A1 bekannt geworden. Dabei wird das die Nadelelektroden enthaltende Elektrodengehäuse mit Hilfe einer Klebefolie an der Hautoberfläche des zu stimulierenden Areals befestigt und mit dem Stimulator über eine Leitung verbunden. Auch der Stimulator kann mit einem Haftkleber in der Nähe des zu stimulierenden Areals an der Hautoberfläche angeklebt oder mit Hilfe eines Nackenbands um den Hals oder um die Schulter angeordnet werden.

Insbesondere bei längeren Behandlungszeiten im Bereich einiger Tage stellt die Befestigung des Stimulators mit Haftkleber ein Problem dar, da die Befestigung nach dem Duschen erneuert werden muss.

Das Ziel der vorliegenden Erfindung ist die Schaffung eines oben genannten elektrischen Stimulationsgeräts bei dem die erwähnten Nachteile vermieden oder zumindest reduziert werden können und eine einfachere Befestigung des Stimulators an der Hautoberfläche in der Nähe des zu stimulierenden Areals ermöglicht werden kann.

Gelöst wird die erfindungsgemäße Aufgabe durch ein oben genanntes elektrisches Stimulationsgerät, wobei das Befestigungselement eine Spange zur lösbaren Aufnahme des Stimulatorgehäuses und ein Auflageelement zur Auflage und Befestigung des Stimulators an der Hautoberfläche aufweist. Durch die lösbare Verbindung des Stimulators mit einem Befestigungselement kann eine Trennung der eigentlichen Stimulationsfunktion des Stimulators von der Befestigungsfunktion erzielt werden. Dadurch, dass der Stimulator lösbar mit dem Befestigungselement verbindbar ist, können in einem Set mehrere Befestigungselemente zur Verfügung gestellt werden, sodass der Stimulator des Stimulationsgeräts auch mehrfach während der Behandlungsdauer an der Hautoberfläche befestigt werden kann. Das Befestigungselement kann im Vergleich zum Stimulator wesentlich kostengünstiger hergestellt werden. Eine entsprechende Konstruktion des Befestigungselements vorausgesetzt, kann eine rasche, einfache und sichere Befestigung an der Hautoberfläche an einer gewünschten Stelle vorgenommen werden. Die Spange des Befestigungselements umgreift das Stimulatorgehäuse und nimmt dieses im Betrieb des elektrischen Stimulationsgeräts auf. Vorzugsweise erfolgt die Verbindung des Stimulatorgehäuses mit der Spange des Befestigungselements werkzeuglos und ohne Zuhilfenahme von zusätzlichen Hilfsmitteln. Das Auflageelement des Befestigungselements dient zur Auflage und Befestigung des Stimulators an der Hautoberfläche. Je nach Ausführungsform und gewünschter Stelle der Befestigung des Stimulators kann eine Befestigung mit Hilfe eines doppelseitigen Klebebandes, einer Klebefolie oder auch eines elastischen Bandes mit einem Schnellverschluss, insbesondere Klettverschluss, vorgenommen werden.

Insbesondere bei der Befestigung mit Hilfe eines Klebers ist es von Vorteil, wenn die Spange des Befestigungselements im Wesentlichen 90° zum Auflageelement orientiert ist. Durch diese winkelige Anordnung der Spange im Bezug auf das Auflageelement des Befestigungselements wird verhindert, dass die Befestigung des Auflageelements an der Hautoberfläche beim Verbinden und Lösen des Stimulators von der Spange negativ beeinflusst wird.

Gemäß einem weiteren Merkmal der Erfindung weist die Spange des Befestigungselements Rastelemente zum Einrasten in zumindest eine Nut am Stimulatorgehäuse auf. Eine derartige Realisierung der lösbaren Verbindung zwischen Stimulatorgehäuse und Befestigungselement ist einfach und kostengünstig herstellbar und erfordert keine zusätzlichen Hilfsmittel zur Herstellung und Lösung der Verbindung zwischen Stimulatorgehäuse und Befestigungselement. Natürlich kann auch die Spange eine Nut aufweisen und am Stimulatorgehäuse können entsprechende Rastelemente zum Einrasten in diese Nut an der Spange vorgesehen sein.

Dabei ist es von Vorteil, wenn die zumindest eine Nut asymmetrisch am Stimulatorgehäuse angeordnet ist, sodass das Stimulatorgehäuse nur in einer Orientierung mit dem Befestigungselement verbindbar ist. Auf diese Weise kann verhindert werden, dass der Stimulator verkehrt an der Spange platziert wird.

Vorteilhafterweise sind die zumindest zwei Nadelelektroden in einem gemeinsamen Elektrodengehäuse angeordnet. Dadurch wird einerseits die Produktion vereinfacht, aber auch ein definierter Abstand zwischen den zumindest zwei Nadelelektroden erzielt, welcher für die elektrische Stimulation wesentlich sein kann. Zusätzlich wird das Einstechen der Nadelelektroden in die Hautoberfläche gegenüber einzelnen Nadelelektroden wesentlich vereinfacht. Insbesondere bei der Elektroakupunktur im Bereich des Ohres eines Menschen weisen die Nadelelektroden einen Durchmesser von nur einigen zehntel Millimetern und eine Einstechtiefe von wenigen Millimetern auf, wodurch die Handhabung einzelner Nadelelektroden nahezu unmöglich wäre.

Zur Befestigung des Elektrodengehäuses an der Hautoberfläche ist vorzugsweise eine entsprechend geformte Klebefolie vorgesehen. Diese Klebefolie kann vom Elektrodengehäuse unabhängig vorliegen oder in diesem bereits enthalten sein. Durch übliches Abziehen einer Abdeckfolie wird die Klebeschicht freigegeben und kann dann an der gewünschten Stelle an der Hautoberfläche aufgeklebt werden. Zur Vermeidung von Hautirritationen müssen die Klebestoffe aus entsprechendem bioverträglichem Material hergestellt sein. Eine gewisse Luft- und Feuchtigkeitsdurchlässigkeit der Klebefolie kann von Vorteil sein und die Akzeptanz erhöhen.

Vorteilhafterweise ist auch eine Klebefolie zur Befestigung des Befestigungselements an der Hautoberfläche vorgesehen. Diese Klebefolie ist ebenfalls entsprechend geformt oder bereits am Befestigungselement enthalten. Auf diese Weise erfolgt die Befestigung des Stimulators ebenfalls an der Hautoberfläche.

Vorteilhafterweise weist die Klebefolie eine wesentlich größere Auflagefläche als das Befestigungselement auf und überragt dieses. Dadurch kann eine größere Klebefläche und somit ein sichererer Halt des Stimulators an der Hautoberfläche erzielt werden.

Um eine Trennung der Nadelelektroden vom Stimulator erzielen zu können und beispielsweise den Stimulator während des Duschens abnehmen zu können, ist es von Vorteil wenn die Leitung mit zumindest einem Stecker zur lösbaren Verbindung mit einer entsprechenden Buchse am Stimulator oder Elektrodengehäuse versehen ist. Dadurch kann der Patient oder ein Betreuer das Elektrodengehäuse einfach vom Stimulator trennen und wieder verbinden. Die Leitung kann fix mit dem Elektrodengehäuse verbunden und der Stecker am Ende der Leitung befestigt und der Stimulator mit einer entsprechenden Buchse ausgestattet sein. Ebenso ist es möglich, die Leitung fix mit dem Stimulator zu verbinden und den Stecker am Ende der Leitung und die zugehörige Buchse am Elektrodengehäuse anzuordnen.

Der Stecker ist vorzugsweise wasserdicht ausgebildet, um ein Duschen bei befestigten Nadelelektroden auch bei verbundenem und befestigtem Stimulator zu ermöglichen.

Wenn an der der Hautoberfläche zugewandten Seite des Elektrodengehäuses zwischen den zumindest zwei Nadelelektroden eine Kerbe angeordnet ist, kann ein Kurzschluss zwischen den Nadelelektroden durch Schweißbildung wirkungsvoll verhindert werden und somit eine Fehlfunktion des Stimulationsgeräts ausgeschlossen oder zumindest deren Wahrscheinlichkeit reduziert werden.

Um dem Patienten oder Arzt ein Feedback über die Funktion des Stimulators geben zu können, ist am Stimulator vorzugsweise eine Betriebsanzeige angeordnet. Je nach Größe des Stimulators kann diese Betriebsanzeige beispielsweise durch eine oder mehrere Leuchtdioden, aber auch Anzeigen gebildet sein. Neben einer optischen Betriebsanzeige kann auch eine akustische Anzeige des ordnungsgemäßen Betriebs bzw. der ordnungsgemäßen Inbetriebnahme erfolgen.

Wenn am Stimulator ein Bedienungselement angeordnet ist, können beispielsweise die Stimulationsparameter geändert werden.

Dabei kann das Bedienungselement durch einen über einen Magneten betätigbaren Hallsensor gebildet sein. Dadurch kann verhindert werden, dass der Patient unabsichtlich oder absichtlich Änderungen der Stimulationsparameter oder eine Deaktivierung des Stimulators vornimmt. Eine wasserdichte Ausbildung des Stimulators wird durch eine solche Realisierung des Bedienungselements ebenfalls erleichtert. Mit Hilfe einer derartigen berührungslosen Bedienung kann aber beispielsweise der behandelnde Arzt Veränderungen vornehmen. Ein entsprechendes Feedback kann durch optische und oder akustische Signale ausgegeben werden.

Vorteilhafterweise enthält der Stimulator einen Timer zur automatischen Festlegung der Dauer und eventuell auch des Musters der Stimulation. Nach Ablauf der gewünschten Behandlungsdauer und des gewünschten Behandlungsmusters schaltet das Gerät selbstständig ab.

Anhand der beigefügten Zeichnungen wird die vorliegende Erfindung näher erläutert. Darin zeigen:
Fig. 1 ein schematisches Blockschaltbild einer Ausführungsform eines elektrischen Stimulationsgeräts;
Fig. 2 eine Ausführungsform eines Elektrodengehäuses mit zwei Nadelelektroden und einer Leitung zur Verbindung mit dem Stimulator;
Fig. 3 eine weitere Ausführungsform eines Elektrodengehäuses mit zwei Nadelelektroden und einer Leitung zur Verbindung mit dem Stimulator;
Fig. 4 eine Ausführungsform einer Clip-Befestigung des Stimulators in getrennter Form;
Fig. 5 die Ausführungsform der Clip-Befestigung des Stimulatorsgemäß Fig. 4 in verbundener Form;
Fig. 6 ein Blockschaltbild einer Ausführungsform des Stimulators;
Fig. 7 die Beschaltung des Steckers an der Leitung der Nadelelektroden; und
Fig. 8 eine Unteransicht auf eine Ausführungsform des Elektrodengehäuses.

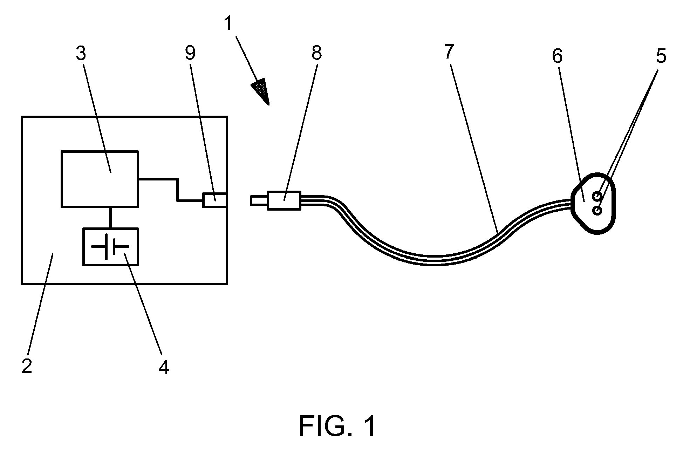

Fig. 1 zeigt ein elektrisches Stimulationsgerät 1, insbesondere zur Stimulation im Bereich des Ohres eines Menschen. Das elektrische Stimulationsgerät 1 beinhaltet einen Stimulator 2 enthaltend einen Generator 3 zur Erzeugung elektrischer Stimulationsimpulse mit bestimmten Stimulationsparametern, d.h. bestimmter Spannung oder bestimmten Stromes, bestimmter Dauer, bestimmter Wiederholfrequenz und Tastverhältnis etc.. Zur Versorgung der Komponenten des Stimulators 2 mit elektrischer Energie ist eine Spannungsversorgung 4 vorgesehen, welche vorzugsweise durch eine entsprechende Batterie oder einen Akkumulator gebildet ist. Die im Generator 3 des Stimulators 2 gebildeten elektrischen Impulse werden über zumindest zwei Nadelelektroden 5, welche in dem zu stimulierenden Areal in die Hautoberfläche eingestochen werden, abgegeben. Dabei sind zumindest eine Masseelektrode und zumindest eine Stimulationselektrode in einem gemeinsamen Elektrodengehäuse 6 angeordnet. Über eine entsprechende Leitung 7 werden die Nadelelektroden 5 mit dem Stimulator 2 verbunden. Durch die gemeinsame Anordnung sämtlicher Nadelelektroden 5 in dem gemeinsamen Elektrodengehäuse 6 wird eine einfache Anwendung ermöglicht, ein besserer Halt der Nadelelektroden 5, insbesondere bei längeren Therapieintervallen, und auch ein definierter Abstand zwischen den einzelnen Nadelelektroden 5 erzielt. Die Leitung 7 zur Verbindung der Nadelelektroden 5 mit dem Stimulator 2 kann fix, d.h. untrennbar, oder auch lösbar angeordnet sein. Zur einfachen Trennung und Verbindung ist es von Vorteil, wenn an der Leitung 7 zumindest ein entsprechender Stecker 8 angeordnet ist. Je nachdem ob die Leitung 7 fix mit dem Elektrodengehäuse 6 oder dem Stimulator 2 verbunden ist, befindet sich die zum Stecker 8 zugehörige Buchse 9 am Stimulator 2 oder im Elektrodengehäuse 6 (nicht dargestellt). Wenn die Leitung 7 als Spiralkabel ausgebildet ist, kann ein Längenausgleich zwischen Elektrodengehäuse 6 und Stimulator 2 und eine bessere Beweglichkeit erzielt werden.

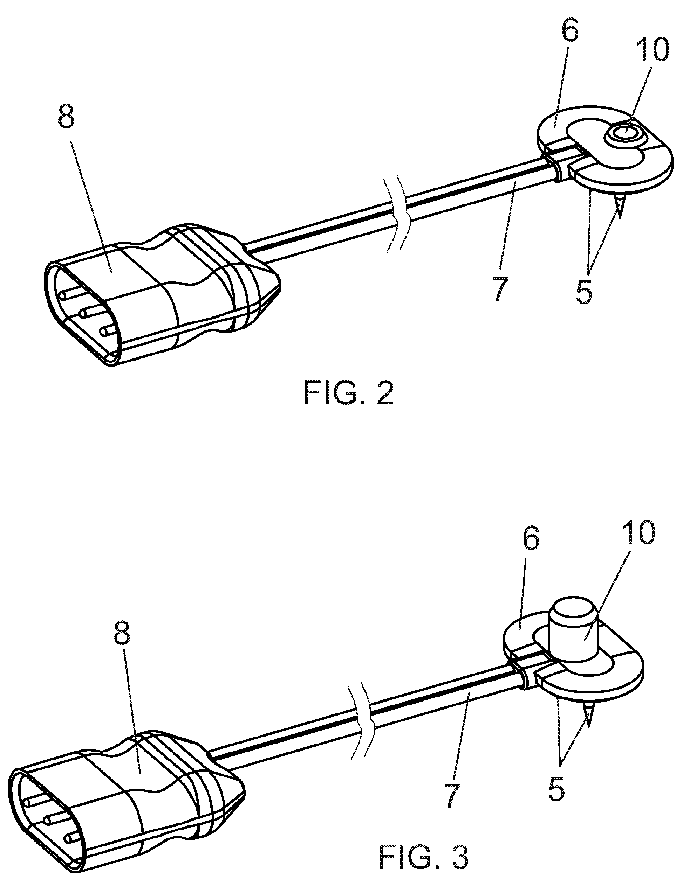

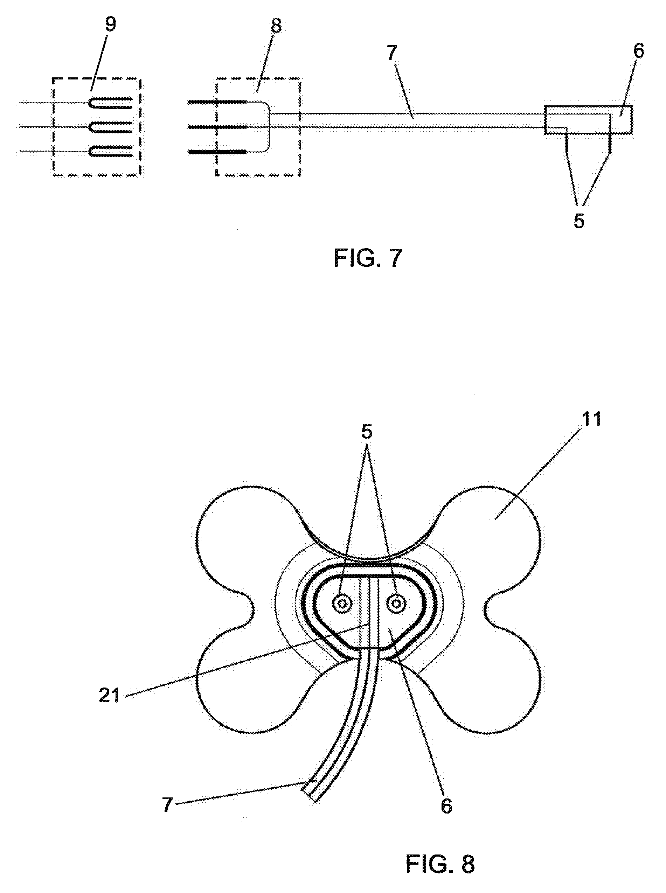

Fig. 2 und 3 zeigen zwei Ausführungsformen eines Elektrodengehäuses 6 mit darin angeordneten Nadelelektroden 5 und fest verbundener Leitung 7 mit daran angeordnetem Stecker 8. Das Elektrodengehäuse 6, welches vorzugsweise aus Kunststoff und vorzugsweise wasserdicht ausgebildet ist, kann an der den Nadelelektroden 5 gegenüberliegenden Seite ein Handhabungselement 10 aufweisen, durch das ein Applizieren der Nadelelektroden 5 an dem gewünschten zu stimulierenden Hautareal erleichtert wird. Das Handhabungselement kann beispielsweise durch eine Vertiefung (Fig. 2) oder einen im Wesentlichen zylindrischen Griff (Fig. 3) gebildet sein. Im dargestellten Ausführungsbeispiel weist der Stecker 8 drei Kontakte auf, obgleich nur zwei Nadelelektroden 5 vorgesehen sind. Durch diese Anordnung, welche anhand von Fig. 7 näher erläutert wird, kann ein automatisches Aktivieren des elektrischen Stimulationsgeräts 1 beim Anstecken der Nadelelektroden 5 am Stimulator 2 erreicht werden.

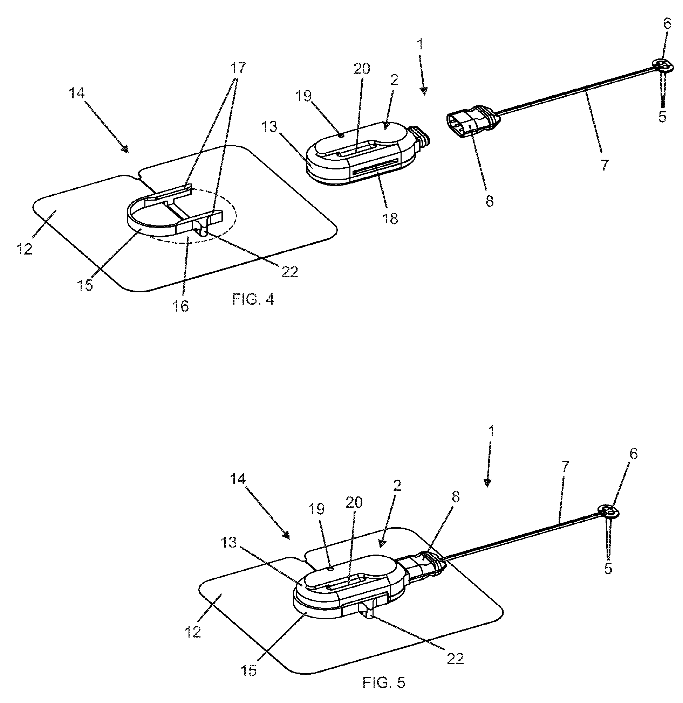

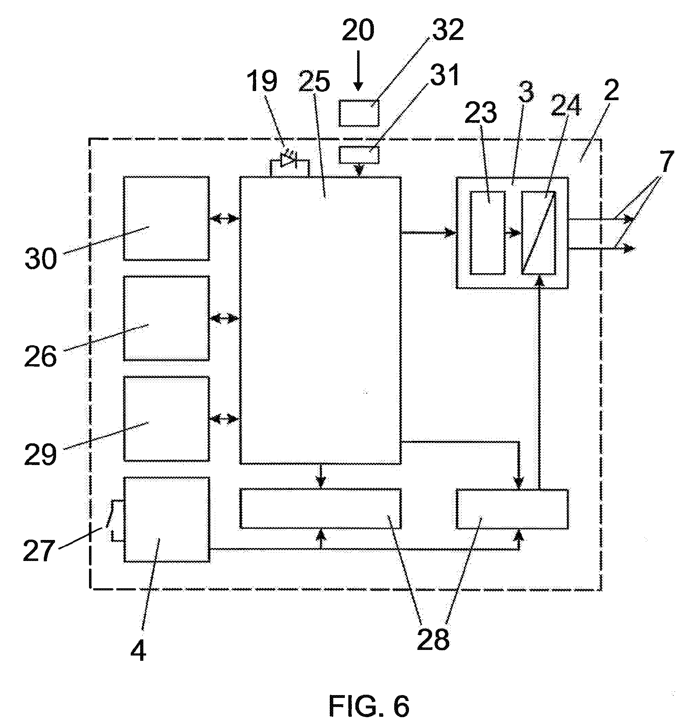

Anhand der Fig. 4 und 5 wird eine Ausführungsform einer erfindungsgemäßen lösbaren Befestigung des Stimulators 2 an einem Befestigungselement 14 erläutert. Bei der Darstellung gemäß Fig. 4 ist der Stimulator 2 des elektrischen Stimulationsgeräts 1 von den Nadelelektroden 5 und vom Befestigungselement 14 getrennt dargestellt, wohingegen das elektrische Stimulationsgerät 1 gemäß Fig. 5 im zusammengebauten Zustand, d.h. mit verbundenen Nadelelektroden 5 und verbundenem Befestigungselement 14, dargestellt ist. Erfindungsgemäß ist der Stimulator 2 lösbar mit dem Befestigungselement 14 verbindbar. Das Befestigungselement 14 weist dazu eine Spange 15 zur lösbaren Aufnahme des Stimulatorgehäuses 13 des Stimulators 2 und ein mit der Spange 15 verbundenes Auflageelement 16 (strichliert dargestellt) zur Auflage und Befestigung des Stimulators 2 an der Hautoberfläche auf. Die Spange 15 und das Auflageelement 16 des Befestigungselements 14 können sehr kostengünstig aus Kunststoff beispielsweise im Spritzgussverfahren hergestellt werden. Die Befestigung an der Hautoberfläche kann über das Auflageelement 16 direkt erfolgen, indem die Unterseite des Auflageelements 16 mit einer Klebeschicht versehen ist. Alternativ dazu kann die Befestigung des Auflageelements 16 auch mit einer entsprechend geformten Klebefolie 12 erfolgen. Die Klebefolie 12 weist an der Unterseite eine abziehbare Abdeckfolie (nicht dargestellt) auf, welche vor der Anwendung abgezogen wird, sodass die Klebeschicht freigelegt wird, wie es bei einem Pflaster üblich ist. Die Spange 15 des Befestigungselements 14 ist vorzugsweise im Wesentlichen 90° zum Auflageelement 16 angeordnet, um zu verhindern, dass bei Trennung des Stimulators 2 vom Befestigungselement 14 das Auflageelement 16 von der Hautoberfläche gezogen wird. Die Spange 15 weist Rastelemente 17 zum Einrasten in zumindest eine Nut 18 am Stimulatorgehäuse 13 auf. Die Nut 18 ist am Stimulatorgehäuse 13 asymmetrisch angeordnet, um ein Einschieben des Stimulators 2 in die Spange 15 nur in einer Orientierung zu ermöglichen. Dadurch wird gewährleistet, dass eine allfällige Betriebsanzeige 19 und ein allfälliges Bedienungselement 20 am Stimulator 2 zugänglich sind. Das Bedienungselement 20 kann berührungslos ausgebildet sein, indem im Stimulator 2 ein Hallsensor angeordnet wird, welcher durch einen Magneten betätigt werden kann (s. Fig. 6). Zur Erleichterung der Trennung des Stimulators 2 vom Befestigungselement 14 kann an der Spange 15 ein Hebel 22 angeordnet sein, über den die Schenkel der Spange 15 auseinandergebogen und die Rastelemente 17 aus der Nut 18 gehoben werden können.

Ein entsprechend ausgestaltetes elektrisches Stimulationsgerät 1 kann auch mit mehreren Befestigungselementen 14 ausgeliefert und vertrieben werden, um ein mehrfaches Befestigen des Stimulators 2 an der Hautoberfläche des Patienten während der Behandlungsdauer von beispielsweise mehreren Tagen zu ermöglichen. Dadurch, dass das Befestigungselement 14 zusammen mit der Klebefolie 12 relativ kostengünstig hergestellt werden kann und vom Stimulator 2 getrennt ist, können mehrere derartige Befestigungselemente 14 einem Set beigefügt werden, ohne den Kaufpreis des elektrischen Stimulationsgeräts 1 wesentlich erhöhen zu müssen.

Fig. 6 zeigt ein Blockschaltbild einer Ausführungsform eines Stimulators 2 des elektrischen Stimulationsgeräts 1. Der Generator 3 zur Erzeugung elektrischer Stimulationsimpulse ist dabei durch einen Digital-Analog-Wandler 23 und einen Spannungs-StromWandler 24 gebildet. Über die Leitungen 7 werden die Nadelelektroden 5 (nicht dargestellt) mit dem Stimulator 2 verbunden. Die Spannungsversorgung 4 ist vorzugsweise durch eine Batterie gebildet. Über eine Steuereinrichtung 25 erfolgt die Ansteuerung des Generators 3 und die Festlegung der Stimulationsparameter und Steuerung des Stimulationsablaufs. Der Generator 3 des Stimulators 2 ist vorzugsweise zur Erzeugung von Stimulationsimpulsen mit einer Wiederholfrequenz von 0,5 bis 100 Hz, vorzugsweise 1 Hz und einem Tastverhältnis von 10 bis 90 %, vorzugsweise 50% ausgebildet. Derartige Stimulationsparameter haben sich bei Anwendungen in der Schmerztherapie oder bei der Wundheilung als bevorzugt herausgestellt. Da die Hautimpedanz große Schwankungen aufweist, ist es von Vorteil, wenn die Stimulationsimpulse mit konstanter Stromamplitude erzeugt werden. Die Steuereinrichtung 25 ist vorzugsweise durch einen entsprechenden Mikrokontroller gebildet. Über eine Schnittstelle 26 kann die Steuereinrichtung 25 programmiert bzw. deren Parameter geändert werden. Eine Änderung der Stimulationsparameter kann auch durch entsprechende Bedienungselemente 20, welche mit der Steuereinrichtung 25 verbunden sind, von außen manuell vorgenommen werden. Die Bedienungselemente 20 können beispielsweise durch einen entsprechenden Hallsensor 31 gebildet sein, der mit der Steuereinrichtung 25 verbunden ist und über einen Magnet 32 betätigt werden kann. Beispielsweise kann die Stromamplitude der Stimulationsimpulse über das Bedienungselement 20 eingestellt und verändert werden. Über einen Schalter 27 kann eine Aktivierung des Stimulators 2 bzw. der darin vorgesehenen Spannungsversorgung 4 vorgenommen werden. Vorteilhafterweise wird aber auf einen derartigen Schalter 27 verzichtet und die Aktivierung des Stimulators 2 automatisch beim Anstecken der Nadelelektroden 5 an den Stimulator 2 vorgenommen. Um zu erzielen, dass mit den Nadelelektroden 5 Stimulationsimpulse mit konstanter Stromamplitude abgegeben werden können, sind mit der Steuereinrichtung 25 entsprechende Regler 28 verbunden, die wiederum mit dem Spannungsstromwandler 24 verbunden sind.

Weiters kann mit der Steuereinrichtung 25 ein Timer 29 verbunden sein, der die Stimulationsdauer und das Stimulationsmuster festlegt. Wenn der Stimulator 2 einen Speicher 30 aufweist, können die Stimulationsparameter in Abhängigkeit der Zeit für nachträgliche Untersuchungen oder zur Qualitätskontrolle aufgezeichnet werden. Der Speicher 30 kann über die Schnittstelle 26 durch einen entsprechenden Computer ausgelesen werden.

Zur Anzeige des ordnungsgemäßen Betriebs des Stimulationsgeräts 1 bzw. Stimulators 2 kann eine entsprechende Betriebsanzeige 19 vorgesehen sein, die beispielsweise durch eine Leuchtdiode realisiert werden kann.

Fig. 7 zeigt das Prinzip der Beschaltung des Steckers 8 an der Leitung 7 der Nadelelektroden 5, welche bereits oben anhand der Figuren 2 und 3 kurz erwähnt wurde. Demgemäß weist der Stecker 8 drei Kontakte auf, von denen die beiden äußeren mit einer Nadelelektrode 5 und der mittlere mit der anderen Nadelelektrode 5 verbunden ist. Auf diese Weise kann zusammen mit einer entsprechenden (nicht dargestellten) Beschaltung der Buchse 9 ein automatisches Aktivieren des Stimulators 2 beim Verbinden des Steckers 8 mit der Buchse 9 erreicht werden, da über die äußeren Kontakte des Steckers 8 ein Verbinden bzw. Schalten erzielt werden kann.

Schließlich zeigt Fig. 8 eine Unteransicht auf eine Ausführungsform eines Elektrodengehäuses 6 mit zwei Nadelelektroden 5. Zwischen den Nadelelektroden 5 ist eine Kerbe 21 angeordnet, welche einen Kurzschluss zwischen den Nadelelektroden 5 beispielsweise durch Schweiß verhindert bzw. dessen Bildung erschwert und somit Fehlfunktionen des Stimulationsgeräts 1 vermeidet. Über eine entsprechend gestaltete Klebefolie 11 kann das Elektrodengehäuse 6 an der Hautoberfläche des Patienten an dem zu stimulierenden Areal befestigt werden. Die Klebefolie 11 weist entsprechend abstehende Laschen auf, um die Klebefläche zu erhöhen und eine sichere Befestigung am entsprechenden zu stimulierenden Areal der Hautoberfläche zu erzielen. Je nachdem welche Körperregion stimuliert werden soll, kann die Klebefolie 11 entsprechend unterschiedlich gestaltet werden.

## Patentansprüche

1. Elektrisches Stimulationsgerät (1) mit einem Stimulator (2), enthaltend einen Generator (3) zur Erzeugung elektrischer Stimulationsimpulse mit bestimmten Stimulationsparametern, eine Spannungsversorgung (4) zur Versorgung des Generators (3) mit elektrischer Energie und ein Stimulatorgehäuse (13), mit einem Befestigungselement (14) zur Befestigung des Stimulators (2) an der Hautoberfläche, mit zumindest zwei Nadelelektroden (5) zum Einstechen in die Hautoberfläche eines zu stimulierenden Areals, mit einer Leitung (7), über welche die Nadelelektroden (5) mit dem Stimulator (2) verbunden sind, wobei der Stimulator (2) lösbar mit dem Befestigungselement (14) verbindbar ist, **dadurch gekennzeichnet, dass** das Befestigungselement (14) eine Spange (15) zur lösbaren Aufnahme des Stimulatorgehäuses (13) und ein Auflageelement (16) zur Auflage und Befestigung des Stimulators (2) an der Hautoberfläche aufweist.

2. Elektrisches Stimulationsgerät (1) nach Anspruch 1, **dadurch gekennzeichnet, dass** die Spange (15) im Wesentlichen 90° zum Auflageelement (16) orientiert ist.

3. Elektrisches Stimulationsgerät (1) nach Anspruch 1 oder 2, **dadurch gekennzeichnet, dass** die Spange (15) Rastelemente (17) zum Einrasten in zumindest eine Nut (18) am Stimulatorgehäuse (13) aufweist.

4. Elektrisches Stimulationsgerät (1) nach Anspruch 3, **dadurch gekennzeichnet, dass** die zumindest eine Nut (18) asymmetrisch am Stimulatorgehäuse (13) angeordnet ist, sodass das Stimulatorgehäuse (13) nur in einer Orientierung mit dem Befestigungselement (14) verbindbar ist.

5. Elektrisches Stimulationsgerät (1) nach einem der Ansprüche 1 bis 4, **dadurch gekennzeichnet, dass** die zumindest zwei Nadelelektroden (5) in einem gemeinsamen Elektrodengehäuse (6) angeordnet sind.

6. Elektrisches Stimulationsgerät (1) nach Anspruch 5, **dadurch gekennzeichnet, dass** eine Klebefolie (11) zur Befestigung des Elektrodengehäuses (6) an der Hautoberfläche vorgesehen ist.

7. Elektrisches Stimulationsgerät (1) nach einem der Ansprüche 1 bis 6, **dadurch gekennzeichnet, dass** eine Klebefolie (12) zur Befestigung des Befestigungselements (14) an der Hautoberfläche vorgesehen ist.

8. Elektrisches Stimulationsgerät (1) nach Anspruch 7, **dadurch gekennzeichnet, dass** die Klebefolie (12) eine wesentlich größere Auflagefläche als das Befestigungselement (14) aufweist und dieses überragt.

9. Elektrisches Stimulationsgerät (1) nach einem der Ansprüche 1 bis 8, **dadurch gekennzeichnet, dass** die Leitung (7) mit zumindest einem Stecker (8) zur lösbaren Verbindung mit einer entsprechenden Buchse (9) am Stimulator (2) oder Elektrodengehäuse (6) versehen ist.

10. Elektrisches Stimulationsgerät (1) nach Anspruch 9, **dadurch gekennzeichnet, dass** der zumindest eine Stecker (8) wasserdicht ausgebildet ist.

11. Elektrisches Stimulationsgerät (1) nach einem der Ansprüche 5 bis 10, **dadurch gekennzeichnet, dass** an der der Hautoberfläche zugewandten Seite des Elektrodengehäuses (6) zwischen den zumindest zwei Nadelelektroden (5) eine Kerbe (21) angeordnet ist.

12. Elektrisches Stimulationsgerät (1) nach einem der Ansprüche 1 bis 11, **dadurch gekennzeichnet, dass** am Stimulator (2) eine Betriebsanzeige (19) angeordnet ist.

13. Elektrisches Stimulationsgerät (1) nach einem der Ansprüche 1 bis 12, **dadurch gekennzeichnet, dass** am Stimulator (2) ein Bedienungselement (20) angeordnet ist.

14. Elektrisches Stimulationsgerät (1) nach Anspruch 13, **dadurch gekennzeichnet, dass** das Bedienungselement (20) durch einen über einen Magneten (32) betätigbaren Hallsensor (31) gebildet ist.

15. Elektrisches Stimulationsgerät (1) nach einem der Ansprüche 1 bis 14, **dadurch gekennzeichnet, dass** der Stimulator (2) einen Timer (29) zur automatischen Festlegung der Dauer der Stimulation enthält.

## Claims

1. An electrical stimulation device (1) having a stimulator (2), containing a generator (3) for generating electrical stimulation pulses having specific stimulation parameters, a voltage supply (4) for supplying the generator (3) with electrical energy and a stimulator housing (13), having a fastening element (14) for fastening the stimulator (2) to the surface of the skin, having at least two needle electrodes (5) for piercing the surface of the skin of an area to be stimulated, having a line (7) via which the needle electrodes (5) are connected to the stimulator (2), wherein the stimulator (2) can be releasably connected to the fastening element (14), **characterised in that** the fastening element (14) has a clip (15) for releasably receiving the stimulator housing (13) and a support element (16) for supporting and fastening the stimulator (2) to the surface of the skin.

2. The electrical stimulation device (1) according to claim 1, **characterised in that** the clip (15) is oriented at substantially 90° to the support element (16).

3. The electrical stimulation device (1) according to claim 1 or 2, **characterised in that** the clip (15) has latching elements (17) for snapping into at least one groove (18) in the stimulator housing (13).

4. The electrical stimulation device (1) according to claim 3, **characterised in that** the at least one groove (18) is disposed asymmetrically on the stimulator housing (13) so the stimulator housing (13) may only be connected to the fastening element (14) in one orientation.

5. The electrical stimulation device (1) according to any one of claims 1 to 4, **characterised in that** the at least two needle electrodes (5) are disposed in a common electrode housing (6).

6. The electrical stimulation device (1) according to claim 5, **characterised in that** an adhesive foil (11) is provided for attaching the electrode housing (6) to the surface of the skin.

7. The electrical stimulation device (1) according to any one of claims 1 to 6, **characterised in that** an adhesive foil (12) is provided for attaching the fastening element (14) to the surface of the skin.

8. The electrical stimulation device (1) according to claim 7, **characterised in that** the adhesive foil (12) has a much larger contact area than the fastening element (14) and projects over it.

9. The electrical stimulation device (1) according to any one of claims 1 to 8, **characterised in that** the line (7) is provided with at least one plug (8) for releasably connecting it to a corresponding jack (9) in the stimulator (2) or the electrode housing (13).

10. The electrical stimulation device (1) according to claim 9, **characterised in that** the at least one plug (8) is designed waterproof.

11. The electrical stimulation device (1) according to any one of claims 5 to 10, **characterised in that** a notch (21) is disposed between the at least two needle electrodes (5) on the side of the electrode housing (6) facing the surface of the skin.

12. The electrical stimulation device (1) according to any one of claims 1 to 11, **characterised in that** an operation indicator (19) is provided on the stimulator (2).

13. The electrical stimulation device (1) according to any one of claims 1 to 12, **characterised in that** a control element (20) is provided on the stimulator (2).

14. The electrical stimulation device (1) according to claim 13, **characterised in that** the control element (20) is formed by a Hall sensor (31) that may be actuated by a magnet (32).

15. The electrical stimulation device (1) according to any one of claims 1 to 14, **characterised in that** the stimulator (2) has a timer (29) for automatically specifying the duration of stimulation.

## Revendications

1. Appareil de stimulation électrique (1) avec un stimulateur (2) comprenant un générateur (3) pour la production d'impulsions de stimulation électrique avec des paramètres de stimulation déterminées, une alimentation en tension (4) pour l'alimentation du générateur (3) en énergie électrique et un boîtier de stimulateur (13) avec un élément de fixation (14) pour la fixation du stimulateur (2) sur la surface de la peau, avec au moins deux électrodes aiguilles (5) à enfoncer dans la surface de la peau d'une zone à stimuler, avec une ligne (7) par l'intermédiaire de laquelle les électrodes aiguilles (5) sont reliées avec le stimulateur (2), le stimulateur (2) pouvant être relié de manière amovible avec l'élément de fixation (14), **caractérisé en ce que** l'élément de fixation (14) comprend une agrafe (15) pour le logement amovible du boîtier de stimulateur (13) et un élément d'appui (16) pour l'appui et la fixation du stimulateur (2) sur la surface de la peau.

2. Appareil de stimulation électrique (1) selon la revendication 1, **caractérisé en ce que** l'agrafe (15) est orientée globalement de 90° par rapport à l'élément d'appui (16).

3. Appareil de stimulation électrique (1) selon la revendication 1 ou 2, **caractérisé en ce que** l'agrafe (15) comprend des éléments d'encliquetage (17) destinés à être encliquetés dans au moins une rainure (18) sur le boîtier de stimulateur (13).

4. Appareil de stimulation électrique (1) selon la revendication 3, **caractérisé en ce que** l'au moins une rainure (18) est disposée de manière asymétrique sur le boîtier de stimulateur (13), de façon à ce que le boîtier de stimulateur (13) puisse être relié uniquement avec une orientation déterminée par rapport à l'élément de fixation (14).

5. Appareil de stimulation électrique (1) selon l'une des revendications 1 à 4, **caractérisé en ce que** les au moins deux électrodes aiguilles (5) sont disposées dans un boîtier d'électrodes (6) commun.

6. Appareil de stimulation électrique (1) selon la revendication 5, **caractérisé en ce qu'**un film adhésif (11) est prévu pour la fixation du boîtier d'électrode (6) sur la surface de la peau.

7. Appareil de stimulation électrique (1) selon l'une des revendications 1 à 6, **caractérisé en ce qu'**un film adhésif (12) est prévu pour la fixation de l'élément de fixation (14) sur la surface de la peau.

8. Appareil de stimulation électrique (1) selon la revendication 7, **caractérisé en ce que** le film adhésif (12) présente une surface d'appui nettement supérieure à l'élément de fixation (14) et dépasse de celui-ci.

9. Appareil de stimulation électrique (1) selon l'une des revendications 1 à 8, **caractérisé en ce que** la ligne (7) est munie d'au moins un connecteur (8) pour la liaison amovible avec une prise (9) correspondante sur le stimulateur (2) ou le boîtier d'électrode (6).

10. Appareil de stimulation électrique (1) selon la revendication 9, **caractérisé en ce que** l'au moins un connecteur (8) est conçu de manière étanche à l'eau.

11. Appareil de stimulation électrique (1) selon l'une des revendications 5 à 10, **caractérisé en ce que**, sur le côté du boîtier d'électrode (6) orienté vers la surface de la peau, entre les au moins deux électrodes aiguilles (5), se trouve une encoche (21).

12. Appareil de stimulation électrique (1) selon l'une des revendications 1 à 11, **caractérisé en ce que**, sur le stimulateur (2), se trouve un affichage de fonctionnement (19).

13. Appareil de stimulation électrique (1) selon l'une des revendications 1 à 12, **caractérisé en ce que**, sur le stimulateur (2), se trouve un élément de commande (20).

14. Appareil de stimulation électrique (1) selon la revendication 13, **caractérisé en ce que** l'élément de commande (20) est constitué d'un capteur à effet Hall (31) actionnable par l'intermédiaire d'un aimant (32).

15. Appareil de stimulation électrique (1) selon l'une des revendications 1 à 14, **caractérisé en ce que** le stimulateur (2) comprend une minuterie (29) pour la détermination automatique de la durée de la stimulation.
